# EUROPÄISCHE PATENTANMELDUNG

(11) **EP 3 888 759 A1**
(43) Veröffentlichungstag der Anmeldung: **06.10.2021**
(21) Anmeldenummer: 21151720.6
(22) Anmeldetag: 15.01.2021
(51) Int. Cl.: A62B 18/02, A61L 9/20, A41D 13/11

(54) **ATEMLUFTSTERILISIERUNGSEINRICHTUNG UND ATEMSCHUTZMASKE**

(30) Priorität: 02.04.2020 DE 202020101798 U
(71) Anmelder: Ldiamon AS, 50411 Tartu (EE)
(72) Erfinder: Frorip, Aleksandr, 61508 Elva (EE); Slivinskiy, Evgeny, 00940 Helsinki (FI)
(74) Vertreter: Kruspig, Volkmar

(57) **Zusammenfassung**

Die Erfindung betrifft eine Atemluftsterilisierungseinrichtung, aufweisend einen Grundkörper, welcher einen langgestreckten Atemluftkanal definiert, dessen Längserstreckung ein Mehrfaches seiner Breite und Höhe beträgt, mindestens eine in den Grundkörper derart eingesetzte UV-LED, die im US-C-Band emittiert, dass die Strahlung der LED einen wesentlichen Teil der Längserstreckung des Grundkörpers durchläuft, und ein Stromversorgungselement der UV-LED.

## Beschreibung

Die Erfindung betrifft eine Atemluftsterilisierungseinrichtung sowie eine Atemschutzmaske, die eine Atemluftsterilisierungseinrichtung umfasst.

Atemschutzmasken sind ein unverzichtbarer Ausrüstungsgegenstand von Kliniken, Quarantänestationen u. ä. und werden in Zeiten von Pandemien auch massenhaft in anderen Bereichen benötigt, um die Ausbreitung von Infektionen durch das Einatmen der verursachenden Viren und Keime zu verhindern.

Herkömmliche Atemschutzmasken enthalten passive Filter, die insbesondere das Einatmen von keimbelasteten Wassertröpfchen und Partikeln verhindern sollen. Um das Atmen der Träger der Atemschutzmasken nicht übermäßig zu erschweren, kann der Porendurchmesser solcher Filter nicht beliebig klein sein, so dass sehr kleine Tröpfchen und Partikel, speziell mit Abmessungen unter einem Mikrometer, nicht ohne weiteres ausgefiltert werden können. Dies würde auch zu einem relativ schnellen Verschließen der Poren und damit zu einem Unbrauchbarwerden der Maske führen.

Daher sind in der Vergangenheit verschiedene Arten von Atemschutzmasken entwickelt worden, die Sterilisierungsmittel zur weitgehenden Keimfreimachung von Tröpfchen und Partikeln dienen, die den passiven Filter der Maske passieren konnten; vgl. hierzu etwa WO 2008/120005 A1, JP 2017/530730 A oder WO 2014/160149 A2.

Der Erfindung liegt die Aufgabe zu Grunde, eine leicht und kostengünstig herzustellende und hochgradig wirksame Atemluftsterilisierungseinrichtung sowie eine entsprechende Atemschutzmaske bereitzustellen.

Diese Aufgabe wird durch eine Atemluftsterilisierungseinrichtung mit den Merkmalen des Anspruchs 1 sowie durch eine Atemschutzmaske mit den Merkmalen des Anspruchs 12 gelöst. Zweckmäßige Fortbildungen des Erfindungsgedankens sind Gegenstand der abhängigen Ansprüche.

Die Erfindung schließt den Gedanken ein, zur Sterilisierung der in den Mund eines Trägers gelangenden Atemluft mindestens eine UV-LED zu nutzen, die UV-Strahlung im sogenannten C-Band emittiert, welche hochgradig keimtötend wirkt, und konstruktiv dafür Sorge zu tragen, dass deren Strahlung über eine relativ lange Strecke auf eine Atemluftströmung einwirkt. Hierzu hat die vorgeschlagene Einrichtung einen Grundkörper, welcher einen langgestreckten Atemluftkanal definiert, dessen Längserstreckung ein Mehrfaches seiner Breite und Höhe beträgt und die UV-LED derart in den Grundkörper eingesetzt ist, dass die Strahlung der LED einen wesentlichen Teil der Längserstreckung des Grundkörpers durchläuft.

Als UV-C-Bereich wird hier insbesondere der Wellenlängenbereich zwischen 250 nm und 315 nm verstanden, wobei als UV-LEDs insbesondere solche eingesetzt werden sollen, deren Emissionswellenlänge im Bereich zwischen 250 nm und 280 nm liegt und die kommerziell verfügbar sind.

Die eine oder mehreren LED/s kann/können insbesondere so in den Atemluftkanal eingebaut sein, dass ihre Strahlung unter einem Winkel von deutlich weniger als 90°, insbesondere von weniger als 45° und noch spezieller von weniger als 30°, auf eine gegenüberliegende, UV-reflektierend ausgestattete Wandung des Kanals trifft, dort teilweise unter einem vergleichbaren Winkel auf einen gegenüberliegenden Wandungsabschnitt reflektiert wird usw., so dass die Strahlung gewissermaßen zickzackförmig den Atemluftkanal durchläuft, wobei eine möglichst hochgradig reflektierende Wandung dafür sorgt, dass die Strahlungsenergie auch nach mehreren Reflexionen noch keimmindernd wirkt.

Es versteht sich, dass zur Stromversorgung der UV-LED weiterhin ein Stromversorgungselement vorgesehen sein muss, und zwar insbesondere in die Sterilisierungseinrichtung integriert.

Die große Längserstreckung des Atemluftkanals, als Voraussetzung einer großen Wirkungslänge der UV-C-Strahlung, wird in Ausführungen der Erfindung dadurch erreicht, dass der Grundkörper einen spiralförmig, schraubenförmig oder schneckenförmig ausgeführten Atemluftkanal umfasst. In einer speziellen Ausgestaltung dieser Ausführung ist der Grundkörper scheibenförmig, mit einem spiralförmig vom Außenumfang der Scheibe nach innen verlaufenden Atemluftkanal, ausgeführt. Grundsätzlich kann der Grundkörper aber auch zylindrisch oder kegelstumpfförmig oder ähnlich konfiguriert sein, um einen schraubenförmig gewendelten oder schneckenförmig konstruierten Atemluftkanal zu beherbergen.

In einer leicht massenhaft herstellbaren Ausführung ist der Grundkörper als Kunststoff-Spritzgussteil gefertigt. Grundsätzlich kann er aber auch aus mehreren Komponenten zusammengesetzt sein, und er muss nicht notwendigerweise aus einem Massenkunststoff bestehen, sondern kann auch aus einem recyclingfähigen Material gefertigt sein.

Als bevorzugt anzusehen sind Ausführungen, bei denen am Einlass und/oder Auslass ein Tröpfchen- und Partikelfilterelement angeordnet ist. Besonders bevorzugt ist hierbei die Anordnung des Filters auf der Eingangsseite.

Des Weiteren ist aus derzeitiger Sicht bevorzugt eine Ausführung, bei der am Einlass oder Auslass ein selbsttätig durch das Lufteinziehen eines Trägers geöffnetes und durch dessen Luftausstoßen geschlossenes Lufteinlassventil vorgesehen ist. Ohne ein solches Ventil ist die vorgeschlagene Atemluftsterilisierungseinrichtung zwar grundsätzlich funktionsfähig, würde aber durch die hochgradig mit großen Tröpfchen belastete ausgeatmete Luft des Trägers relativ schnell verschmutzen und damit graduell ihre Wirksamkeit verlieren.

Speziell bei der oben erwähnten Sterilisierungseinrichtung mit weitgehend scheibenförmigem Grundkörper ist im Mittenbereich der Scheibe ein im Wesentlichen kreisförmiger freier Raum vorgesehen, in dem das Lufteinlassventil und/oder das oder ein Filterelement angeordnet ist. In anderen Ausführungen kann das oder ein Filterelement in den Atemluftkanal vom Einlass oder Auslass her eingeschoben sein.

Zur Gewährleistung einer hohen Effizienz der von der UV-LED emittierten Strahlung ist es vorteilhaft, wenn die Innenwandung des Atemluftkanals UV-C-reflektierend mit einem Reflexionsgrad von mehr als 70 %, insbesondere von mehr als 80 % und noch spezieller von mehr als 90 % ausgeführt ist. Dies ermöglicht es, das Längen-/Querschnitt-Verhältnis des Atemluftkanals in Grenzen zu halten, die einen noch relativ geringen Strömungswiderstand und somit ein relativ leichtes Einatmen der Atemluft ermöglichen.

In einer weiteren, mit der vorbenannten vorteilhaft kombinierbaren Ausführung, weist die Innenwandung des Abluftkanals eine fotokatalytische Beschichtung mit antiviraler Wirkung auf. Hierdurch werden zumindest die an der Wandung entlang streichenden Teil der Atemluft zusätzlich behandelt. Wenn durch die geometrische Konfiguration des Atemluftkanals zudem eine gewisse Verwirbelung des Atemluftstroms bewirkt wird, kann die antivirale Wirkung der fotokatalytischen Beschichtung sogar den überwiegenden Teil des Gesamt-Atemluftstromes erfassen.

Bei der fotokatalytischen Beschichtung kann es sich insbesondere um eine Titandioxid-Beschichtung handeln. Statt der oder zusätzlich zu einer Innenwand-Beschichtung kann im Luftkanal auch ein Volumen-Filterelement aus einem durch die Strahlung der UV-LEDs zu einer antiviralen Wirkung aktivierbaren Material, welches beispielsweise wiederum Titandioxid enthält oder hiermit beschichtet ist, eingesetzt werden.

Weiterhin ist aus derzeitiger Sicht eine Ausführung bevorzugt, bei der in den Grundkörper mehrere UV-C-LEDs derart eingesetzt sind, die jeweils einen Teilbereich der Längserstreckung des Atemluftkanals bestrahlen. Zwar sind solche Ausführungen etwas konstruktions- und kostenaufwändiger als Ausführungen mit einer einzelnen LED, angesichts der akzeptablen Stückpreise der einzusetzenden LEDs kann die erreichbare höhere Wirkung diesen Mehraufwand zumindest in High-End-Ausführungen für besonders problematische Einsatzbereich durchaus rechtfertigen.

Bevorzugt ist weiterhin eine Ausführung, bei der das Stromversorgungselement austausch- oder aufladbar ist. Insbesondere ist dann als Stromversorgungselement ein fest eingebauter Akku vorgesehen und der Grundkörper weist einen Ladeanschluss vom USB-Typ auf. Grundsätzlich kann der Ladeanschluss von einem anderen standardisierten Typ sein, USB-Anschlüsse haben sich aber in den letzten Jahren weitgehend als Akku-Ladeanschlüsse in den verschiedensten Massenprodukten durchgesetzt.

Die vorgeschlagene Atemschutzmaske hat einen Masken-Grundkörper, in den die Atemluftsterilisierungseinrichtung luftdicht eingefügt ist und der zur Abdeckung des unteren Bereiches des Gesichts eines Trägers, einschließlich des Mundes und der Nase und insbesondere einschließlich des Kinns, geformt ist und Haltebänder zum Halten des Masken-Grundkörpers am Kopf und elastisch dichtenden Kantenbereiche, die dazu ausgebildet sind, ein luftdichtes Anliegen der Kanten des Masken-Grundkörpers am Gesicht zu gewährleisten. Grundsätzlich kann ein solcher Maske-Grundkörper nach dem Stand der Technik gefertigt sein, sofern seine Konstruktion das luftdichte Einfügen der vorgeschlagenen Sterilisierungseinrichtung ermöglicht.

Insbesondere kann der Masken-Grundkörper einen flexiblen Flächenkörper umfassen, der insbesondere aus einer Elastomerschicht oder einem luftdicht beschichteten Gewebe gebildet ist. Speziell sollte der flexible Flächenkörper elastische Kantenbereiche aufweisen. Noch spezieller können an den Kantenbereichen des Maskengrundkörpers kompressible und als Tröpfchen- und Partikelfilter wirkende Materialstreifen angebracht sein.

Wenn die Atemschutzmaske eine Ausführung der Sterilisierungseinrichtung umfasst, die - wie oben erwähnt - mit einem Lufteinlassventil ausgestattet ist, muss der Masken-Grundkörper mindestens ein luftdicht in den Masken-Grundkörpers eingefügtes, selbsttätig durch das Luftausstoßen des Trägers geöffnetes und dessen Lufteinziehen geschlossenes Luftauslassventil zum Abführen ausgeatmeter Luft in die Atmosphäre umfassen.

Die Ausführung der Erfindung ist nicht auf die oben erläuterten Ausführungsformen und Aspekte beschränkt, sondern ebenso in einer Vielzahl von Abwandlungen möglich, die im Rahmen fachgemäßen Handelns liegen, insbesondere in beliebigen Kombinationen der Merkmale der anhängenden Ansprüche.

## Patentansprüche

1. Atemluftsterilisierungseinrichtung, aufweisend:
- einen Grundkörper, welcher einen langgestreckten Atemluftkanal definiert, dessen Längserstreckung ein Mehrfaches seiner Breite und Höhe beträgt,
- mindestens eine in den Grundkörper derart eingesetzte UV-LED, die im US-C-Band emittiert, dass die Strahlung der LED einen wesentlichen Teil der Längserstreckung des Grundkörpers durchläuft, und
- ein Stromversorgungselement der UV-LED.

2. Atemluftsterilisierungseinrichtung nach Anspruch 1, wobei der Grundkörper einen spiralförmig, schraubenförmig oder schneckenförmig ausgeführten Atemluftkanal umfasst.

3. Atemluftsterilisierungseinrichtung nach Anspruch 1 oder 2, wobei der Grundkörper scheibenförmig, mit einem spiralförmig vom Außenumfang der Scheibe nach innen verlaufenden Atemluftkanal, ausgeführt ist.

4. Atemluftsterilisierungseinrichtung nach einem der vorangehenden Ansprüche, wobei der Grundkörper als Kunststoff-Spritzgussteil ausgeführt ist.

5. Atemluftsterilisierungseinrichtung nach einem der vorangehenden Ansprüche, wobei am Einlass und/oder Auslass ein Tröpfchen- und Partikelfilterelement angeordnet ist.

6. Atemluftsterilisierungseinrichtung nach einem der vorangehenden Ansprüche, wobei am Einlass oder Auslass ein selbsttätig durch das Lufteinziehen eines Trägers geöffnetes und durch dessen Luftausstoßen geschlossenes Lufteinlassventil vorgesehen ist.

7. Atemluftsterilisierungseinrichtung nach einem der Ansprüche 3 - 5 und Anspruch 6, wobei im Mittenbereich der Scheibe ein im Wesentlichen kreisförmiger freier Raum vorgesehen ist, in dem das Lufteinlassventil und/oder das oder ein Filterelement angeordnet ist.

8. Atemluftsterilisierungseinrichtung nach einem der Ansprüche 5 - 7, wobei das oder ein Tröpfchen- und Partikelfilterelement in den Atemluftkanal vom Einlass oder Auslass her eingeschoben ist.

9. Atemluftsterilisierungseinrichtung nach einem der vorangehenden Ansprüche, wobei die Innenwandung des Atemluftkanals UV-C-reflektierend mit einem Reflexionsgrad von mehr als 70 %, insbesondere von mehr als 80 % und noch spezieller von mehr als 90 % ausgeführt ist und/oder eine fotokatalytische Beschichtung mit antiviraler Wirkung aufweist.

10. Atemluftsterilisierungseinrichtung nach einem der vorangehenden Ansprüche, wobei in den Grundkörper mehrere UV-C-LEDs derart angesetzt sind, dass sie jeweils einen Teilbereich der Längserstreckung des Atemluftkanals bestrahlen.

11. Atemluftsterilisierungseinrichtung nach einem der vorangehenden Ansprüche, wobei insbesondere das Stromversorgungselement austausch- oder aufladbar ist, wobei als Stromversorgungselement ein fest eingebauter Akku vorgesehen ist und der Grundkörper einen Ladeanschluss vom USB-Typ aufweist.

12. Atemschutzmaske, umfassend eine Atemluftsterilisierungseinrichtung nach einem der vorangehenden Ansprüche und weiterhin
einen Masken-Grundkörper, in den die Atemluftsterilisierungseinrichtung luftdicht eingefügt ist und der zur Abdeckung des unteren Bereiches des Gesichts eines Trägers, einschließlich des Mundes und der Nase und insbesondere einschließlich des Kinns, geformt ist und Haltebänder zum Halten des Masken-Grundkörpers am Kopf und elastisch dichtenden Kantenbereiche, die dazu ausgebildet sind, ein luftdichtes Anliegen der Kanten des Masken-Grundkörpers am Gesicht zu gewährleisten.

13. Atemschutzmaske nach Anspruch 12, wobei der Masken-Grundkörper einen flexiblen Flächenkörper umfasst, der insbesondere aus einer Elastomerschicht oder einem luftdicht beschichteten Gewebe gebildet ist.

14. Atemschutzmaske nach Anspruch 13, wobei der flexible Flächenkörper elastische Kantenbereiche aufweist oder an den Kantenbereichen des Maskengrundkörpers kompressible und als Tröpfchen- und Partikelfilter wirkende Materialstreifen angebracht sind.

15. Atemschutzmaske nach einem der Ansprüche 12 - 14, weiterhin umfassend mindestens ein luftdicht in den Masken-Grundkörpers eingefügtes, selbsttätig durch das Luftausstoßen des Trägers geöffnetes und dessen Lufteinziehen geschlossenes Luftauslassventil zum Abführen ausgeatmeter Luft in die Atmosphäre.
